# EUROPEAN PATENT APPLICATION

(11) **EP 4 808 157 A1**
(43) Date of publication of application: **16.09.2026**
(21) Application number: 25163548.8
(22) Date of filing: 13.03.2025
(51) Int. Cl.: H04R 25/00

(54) **METHOD OF OPERATING A HEARING DEVICE TO PROVIDE FOR RELIEVING TINNITUS**

(71) Applicant: Sonova AG, 8712 Stäfa (CH)
(72) Inventor: KNOBLAUCH, Thomas, 8712 Stäfa (CH); BIGGINS, Anna, 8712 Stäfa (CH); STROPAHL, Maren, 8712 Stäfa (CH); HILDEBRAND, Nicola, 8712 Stäfa (CH)

(57) **Abstract**

The disclosure relates to a method of operating a hearing device to provide for relieving tinnitus, the hearing device configured to be worn at an ear of a user, the method comprising receiving an audio stream (SI) from an audio input unit (113); processing the audio stream (SI) in a first audio signal path (P1) into a processed audio stream; and providing an output stream (SO) based on the processed audio stream for an audio output unit (117) included in the hearing device, the audio output unit (117) configured to output the output stream (SO) so as to stimulate the user's hearing. The disclosure also relates to a hearing device for performing the method.

To provide for an improved alleviation of tinnitus symptoms that may also account for a more natural auditory perception, the disclosure proposes processing the audio stream (SI) in a second audio signal path (P2), which outputs a tinnitus masking stream (SM) for relieving tinnitus into the first audio signal path (P1), wherein the second audio signal path (P2) is associated with
evaluating (221, 331) the audio stream (SI) with regard to a presence of one or more preservable characteristics suitable to be preserved in the tinnitus masking stream (SM); and
providing (223, 323) the tinnitus masking stream (SM) based on the preservable characteristics such that one or more of the preservable characteristics of the audio stream (SI) are preserved in the tinnitus masking stream (SM).

## Description

### TECHNICAL FIELD

The disclosure relates to a method of operating a hearing device to provide for relieving tinnitus, according to the preamble of claim 1. The disclosure further relates to a hearing device configured to perform the method, according to the preamble of claim 15.

### BACKGROUND

Hearing devices may be used to improve the hearing capability or communication capability of a user, for instance by compensating a hearing loss of a hearing-impaired user, in which case the hearing device is commonly referred to as a hearing instrument such as a hearing aid, or hearing prosthesis. A hearing device may also be used to output sound based on an audio signal which may be communicated by a wire or wirelessly to the hearing device. A hearing device may also be used to reproduce a sound in a user's ear canal detected by an input transducer such as a microphone or a microphone array. The reproduced sound may be amplified to account for a hearing loss, such as in a hearing instrument, or may be output without accounting for a hearing loss, for instance to provide for a faithful reproduction of detected ambient sound and/or to add audio features of an augmented reality in the reproduced ambient sound, such as in a hearable. A hearing device may also provide for a situational enhancement of an acoustic scene, e.g. beamforming and/or active noise cancelling (ANC), with or without amplification of the reproduced sound. A hearing device may also be implemented as a hearing protection device, such as an earplug, configured to protect the user's hearing. Different types of hearing devices configured to be be worn at an ear include earbuds, earphones, hearables, and hearing instruments such as receiver-in-the-canal (RIC) hearing aids, behind-the-ear (BTE) hearing aids, in-the-ear (ITE) hearing aids, invisible-in-the-canal (IIC) hearing aids, completely-in-the-canal (CIC) hearing aids, cochlear implant systems configured to provide electrical stimulation representative of audio content to a user, a bimodal hearing system configured to provide both amplification and electrical stimulation representative of audio content to a user, or any other suitable hearing prostheses. A hearing system comprising two hearing devices configured to be worn at different ears of the user is sometimes also referred to as a binaural hearing device. A hearing system may also comprise a hearing device, e.g., a single monaural hearing device or a binaural hearing device, and a user device, e.g., a smartphone and/or a smartwatch, communicatively coupled to the hearing device.

Hearing devices are often employed in conjunction with communication devices, such as smartphones or tablets, for instance when listening to sound data processed by the communication device and/or during a phone conversation operated by the communication device. More recently, communication devices have been integrated with hearing devices such that the hearing devices at least partially comprise the functionality of those communication devices. A hearing system may comprise, for instance, a hearing device and a communication device.

Tinnitus is a prevalent auditory condition characterized by the perception of a persistent ringing, buzzing, or hissing sound in the absence of an external acoustic stimulus. This condition affects a significant proportion of the population and can lead to considerable distress, interfering with daily activities, concentration, and sleep quality. In response to this issue, modern hearing instruments have been developed with integrated tinnitus masking functionality. These devices can generate and output masking sounds to alleviate the perceived effects of tinnitus, helping users manage the condition more effectively. Typically, tinnitus masking sounds are selected from a predefined sound library stored in the memory of a mobile phone, which is wirelessly connected to the hearing device. This approach allows users to choose from a variety of pre-recorded sounds to find those that provide the greatest relief.

While this technology represents a meaningful step toward improving the quality of life for tinnitus sufferers, it has several inherent limitations. The number of masking sounds available in a conventional sound library is limited, resulting in repetitive playback of the same sounds over time. This repetitiveness can reduce the effectiveness of tinnitus masking, as users may become accustomed to the sounds, diminishing their ability to provide relief. Moreover, the nature of these pre-recorded sounds does not seamlessly integrate with the user's surrounding environment. Instead, the masking sounds remain distinct from environmental sounds, creating an artificial auditory experience that may feel unnatural and distracting to the user.

Another drawback of conventional tinnitus masking systems is their potential to interfere with speech comprehension. When masking sounds are played at a constant level, they can obscure speech and other important auditory cues in the environment. This issue is particularly pronounced in noisy settings, where understanding speech is already challenging for individuals with hearing loss. In such situations, tinnitus masking sounds can add to the overall auditory complexity, making it difficult for users to engage in conversations and navigate social interactions effectively.

### SUMMARY

It is an object of the present invention to provide for an improved method of operating a hearing device and/or an improved hearing device configured to output tinnitus masking sounds in a manner that overcomes the limitations of the prior art. It is another object to alleviate tinnitus symptoms by offering a more personalized and/or adaptive approach to sound masking. It is a further object to enhance the listening experience by reducing repetitiveness in tinnitus masking. It is a further object to provide for a more natural auditory perception during the tinnitus treatment. It is yet another object to minimize an interference with speech comprehension relative to the tinnitus masking, especially in complex acoustic environments.

At least one of these objects can be achieved by a method of processing an audio signal comprising the features of patent claim 1 and/or a hearing device for performing the method comprising the features of patent claim 15. Advantageous embodiments are defined by the dependent claims and the following description.

Accordingly, the present disclosure proposes of operating a hearing device to provide for a method of relieving tinnitus, the hearing device configured to be worn at an ear of a user, the method comprising
receiving an audio stream from an audio input unit included in the hearing device;
processing the audio stream in a first audio signal path into a processed audio stream;
processing the audio stream in a second audio signal path, which outputs a tinnitus masking stream for relieving tinnitus into the first audio signal path, wherein the second audio signal path is associated with
   evaluating, in an audio stream evaluation operation, the audio stream with regard to a presence of one or more preservable characteristics suitable to be preserved in the tinnitus masking stream; and
   providing, in a tinnitus masking stream provision operation, the tinnitus masking stream based on the preservable characteristics such that one or more of the preservable characteristics of the audio stream are preserved in the tinnitus masking stream; and
   providing an output stream based on the processed audio stream for an audio output unit included in the hearing device, the audio output unit configured to output the output stream so as to stimulate the user's hearing.

Accordingly, an improvement of current tinnitus masking systems can be provided by an evaluation of the audio stream allowing to adapt the tinnitus masking stream to one or more environmental sound characteristics when possible and/or when beneficial for the user, e.g., also with regard to other needs of the user such as a desirable degree of speech comprehension. In some examples, the hearing device may thus be capable of generating custom-made tinnitus masks which may resemble and/or seamlessly blend into the user's environmental soundscape. In some examples, the user's need for a greater variety, adaptability, and/or naturalness of the tinnitus masking may thus be addressed. In some examples, the user can be provided with a more comfortable, distraction-free, and/or effective way to manage tinnitus symptoms.

Independently, the present disclosure proposes a non-transitory computer-readable medium storing instructions that, when executed by a processor, which may be included in a hearing device, cause a hearing device to perform the method.

Independently, the present disclosure proposes a hearing device configured to be worn at an ear of a user, the hearing device comprising an audio input unit for obtaining an audio stream; a processor for audio signal processing of the audio stream to obtain an output stream, wherein the processor is configured to perform the method; and an audio output unit for outputting the output stream so as to stimulate the user's hearing.

Subsequently, additional features of some implementations of the method and/or the hearing device and/or the computer readable medium are described. Each of those features can be provided solely or in combination with at least another feature. The features can be correspondingly provided in some implementations of the method and/or the hearing device and/or the computer readable medium.

In some implementations, the method further comprises modifying the audio stream depending on the evaluated audio stream, wherein the tinnitus masking stream comprises the modified audio stream.

In some implementations, the preservable characteristics comprise a noise and/or a music content in the audio stream, wherein the method further comprises including the noise and/or the music content from the audio stream in the tinnitus masking stream. In some implementations, the method further comprises extracting the noise and/or the music content from the audio stream, wherein the tinnitus masking stream comprises the noise and/or music content. In some implementations, the noise and/or music content from the audio stream is modified before the including in the tinnitus masking stream. E.g., the noise and/or music content may be modified such that an amount and/or regularity and/or level of the noise and/or music content is increased in the tinnitus masking stream.

In some implementations, the method further comprises generating, in a tinnitus masking sound generation operation, a tinnitus masking sound different from the audio stream, wherein the generated tinnitus masking sound is included in the tinnitus masking stream depending on the evaluated audio stream. In some implementations, the tinnitus masking sound is generated based on an artificial noise generated by a noise generator and/or a predetermined masking sound stored in a sound library.

In some implementations, before the generated tinnitus masking sound is included in the tinnitus masking stream, the generated tinnitus masking sound is modified such that one or more of the preservable characteristics of the audio stream are preserved in the tinnitus masking sound. In some implementations, the preservable characteristics of the audio stream preserved in the generated tinnitus masking sound include a timbre of a sound represented by the audio stream; and/or a pitch of a sound represented by the audio stream; and/or a beat and/or rhythm of a sound represented by the audio stream. In some implementations, the generated tinnitus masking sound is modified such that the timbre and/or pitch and/or beat and/or rhythm of sound in the audio stream is preserved in the tinnitus masking sound.

In some implementations, the method further comprises receiving motion data from a motion sensor included in the hearing device, wherein the tinnitus masking sound is generated based on the motion data. In some implementations, the motion data is representative of movements of the user. In some implementations, a number, e.g., a random number, is derived from the motion data, wherein the tinnitus masking sound is generated based on the number. E.g., the number may be input into an artificial noise generator to generate artificial noise with properties depending on the number and/or a masking sound may be selected from a plurality of sounds in a sound library depending on the number.

In some implementations, the noise and/or the music content from the audio stream and the generated tinnitus masking sound are mixed in the tinnitus masking stream according to a mixing ratio, wherein the mixing ratio is determined depending on the evaluated audio stream. In some implementations, the mixing ratio may be provided by a scaling of the noise and/or the music content from the audio stream and/or the generated tinnitus masking sound, which may depend on the evaluated audio stream.

In some implementations, the method further comprises evaluating, in the audio stream evaluation operation, the audio stream with regard to one or more adjustment characteristics representative of an adjustment of the tinnitus masking stream required when the preservable characteristics of the audio stream are preserved in the tinnitus masking stream; and adjusting, in the tinnitus masking stream provision operation, the tinnitus masking stream depending on the adjustment characteristics of the evaluated audio stream.

In some implementations, the adjustment characteristics comprise one or more of a signal to noise ratio of a sound represented by the audio stream; a temporal variation of a sound represented by the audio stream; a level of a sound represented by the audio stream; a presence and/or a type and/or an amount and/or a regularity and/or a level of speech represented by the audio stream; a presence and/or a type and/or an amount and/or a regularity and/or a level of noise represented by the audio stream; a presence and/or a type and/or an amount and/or a regularity and/or a level of music represented by the audio stream; or an acoustic scene attributed to the audio stream by an acoustic scene classifier, wherein one or more of the operations for the processing of the audio stream in the first audio signal path are controlled depending on the acoustic scene attributed to the audio stream. In some implementations, a level and/or frequency and/or type of the tinnitus masking stream is determined depending on the presence and/or type and/or amount and/or regularity and/or volume of speech represented by the audio stream and/or depending on the signal to noise ratio.

In some implementations, the preservable characteristics of the audio stream are adjusted in the tinnitus masking stream depending on the adjustment characteristics. In some implementations, the adjustment characteristics comprise scaling characteristics representative of a scaling of the preservable characteristics required when the preservable characteristics are preserved in the tinnitus masking stream.

In some implementations, the adjustment characteristics comprise adjustment characteristics of the audio stream and/or adjustment characteristics of auxiliary sensor data.

In some implementations, the method further comprises receiving auxiliary sensor data from an auxiliary sensor included in the hearing device, wherein the processing the audio stream in the second audio signal path further comprises evaluating, in an auxiliary sensor data evaluation operation, the auxiliary sensor data with regard to a presence of one or more adjustment characteristics representative of an adjustment of the tinnitus masking stream required when the preservable characteristics of the audio stream are preserved in the tinnitus masking stream; and adjusting, in the tinnitus masking stream provision operation, the tinnitus masking stream depending on the adjustment characteristics of the auxiliary sensor data.

In some implementations, the auxiliary sensor data comprises motion data from a motion sensor and the adjustment characteristics of the motion data comprise one or more of a physical activity of the user; a resting state of the user; a sleeping state of the user; a standing or sitting or lying position of the user; or a walking or running or cycling activity of the user.

In some implementations, the processing in the first audio signal path comprises combining the tinnitus masking stream with the audio stream processed in the first audio signal path such that the output stream is based on the combined stream. In some implementations, the tinnitus masking stream and the audio stream processed in the first audio signal path are mixed the tinnitus masking stream and the audio stream at a mixing ratio. The mixing ration may be determined depending on the evaluated audio stream, e.g., depending on the adjustment characteristics. In some implementations, the mixing ratio is determined depending on a speech contained in the audio stream.

In some implementations, the first audio signal path is associated with applying one or more operations to the audio stream. In some examples, the operations comprise an amplification operation for amplifying the audio stream according to a transfer function. E.g., the transfer function may be adjusted to compensate for a hearing loss of the user. E.g., the amplification of the audio stream may comprise applying a positive gain and/or a negative gain on the audio stream. In some examples, the operations comprise one or more different sound processing programs, e.g., actuators, which are controlled depending on an acoustic scene attributed to the audio stream by an acoustic scene classifier. In some examples, the operations comprise one or more of a beamforming, a gain compression, a frequency compression, a noise cancelling, a feedback cancelling, a wind noise cancelling, an active noise cancelling (ANC), an audio processing performed by a DNN, or any other operation of modifying the audio stream. In some implementations, the second audio signal path at least partially bypasses the first audio signal path.

In some implementations, the audio stream is representative of sound detected in the user's environment. E.g., the audio stream may be representative of environmental sound detected by a microphone included in the hearing device and/or by a remote microphone communicatively coupled with the hearing device, e.g., by an RF receiver.

In some implementations, the method further comprises activating the processing in the second path and/or the tinnitus masking stream depending on a control signal input by the user. E.g., the control signal may be input via a user interface. E.g., the user may be prompted at the user interface about his need for tinnitus relief.

### BRIEF DESCRIPTION OF THE DRAWINGS

Reference will now be made in detail to embodiments, examples of which are illustrated in the accompanying drawings. The drawings illustrate various embodiments and are a part of the specification. The illustrated embodiments are merely examples and do not limit the scope of the disclosure. Throughout the drawings, identical or similar reference numbers designate identical or similar elements. In the drawings:
- Fig. 1: schematically illustrates an exemplary hearing device;
- Fig. 2: schematically illustrates an embodiment of the hearing device illustrated in Fig. 1 as a RIC hearing aid;
- Figs. 3 - 5: schematically illustrate block diagrams of exemplary signal processing algorithms which may be implemented in a hearing device so as to provide for relieving tinnitus; and
- Fig. 6: schematically illustrates an exemplary method of processing an audio signal according to principles described herein.

### DETAILED DESCRIPTION OF THE DRAWINGS

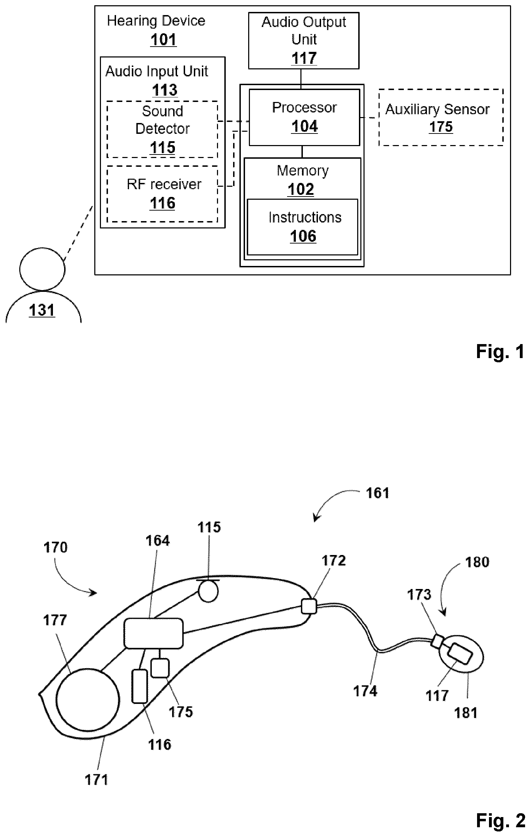

FIG. 1 illustrates an exemplary implementation of a hearing device 101 configured to be worn at an ear of a user 131. Hearing device 101 may be implemented by any type of hearing device configured to enable or enhance hearing or a listening experience of user 131 wearing hearing device 101. For example, hearing device 101 may be implemented by a hearing aid configured to provide an amplified version of audio content to a user, a sound processor included in a cochlear implant system configured to provide electrical stimulation representative of audio content to a user, a sound processor included in a bimodal hearing system configured to provide both amplification and electrical stimulation representative of audio content to a user, an over-the-counter (OTC) hearing device, or any other suitable hearing prosthesis, or an earbud or an earphone or any other hearable.

In certain examples, hearing device 101 may be implemented as part of a binaural hearing system. Such a binaural hearing system may include a first hearing device associated with a first ear of a user and a second hearing device associated with a second ear of a user. In such examples, the hearing devices may each be implemented by any type of hearing device configured to provide or enhance hearing to user 131 of a binaural hearing system. In some examples, the hearing devices in a binaural system may be of the same type. For example, the hearing devices may each be hearing aid devices. In certain alternative examples, the hearing devices may be of a different type. For example, a first hearing device may be a hearing aid and a second hearing device may be a sound processor included in a cochlear implant system.

Different types of hearing device 101 can also be distinguished by the position at which they are worn at the ear. Some hearing devices, such as behind-the-ear (BTE) hearing aids and receiver-in-the-canal (RIC) hearing aids, typically comprise an earpiece configured to be at least partially inserted into an ear canal of the ear, and an additional housing configured to be worn at a wearing position outside the ear canal, in particular behind the ear of the user. Some other hearing devices, as for instance earbuds, earphones, hearables, in-the-ear (ITE) hearing aids, invisible-in-the-canal (IIC) hearing aids, and completely-in-the-canal (CIC) hearing aids, commonly comprise such an earpiece to be worn at least partially inside the ear canal without an additional housing for wearing at the different ear position.

Hearing device 101 may include, without limitation, a memory 102 and a processor 104 selectively and communicatively coupled to one another. Memory 102 and processor 104 may each include or be implemented by hardware and/or software components (e.g., processors, memories, communication interfaces, instructions stored in memory for execution by the processors, etc.).

Memory 102 may maintain (e.g., store) executable data used by processor 104 to perform any of the operations associated with hearing device 101. For example, memory 102 may store instructions 106 that may be executed by processor 104 to perform any of the operations associated with hearing device 101 assisting a user in hearing and/or any of the operations described herein. To illustrate, instructions 106 may include instructions for providing a tinnitus masking scream to provide for relieving tinnitus of user 131. Instructions 106 may further include instructions for providing an amplification of an audio stream. In some examples, instructions 106 may further include audio signal processing routines providing for noise cancelling, feedback cancelling, beamforming, speech enhancement, audio signal classification, e.g., an acoustic scene classification, audio signal processing programs associated with a current acoustic scene, binaural synchronization, and/or the like. Instructions 106 may be implemented by any suitable application, software, firmware, code, and/or other executable data instance.

Processor 104 may be configured to perform any suitable processing operation associated with hearing device 101. For example, when hearing device 101 is implemented by a hearing instrument, such processing operations may include monitoring ambient sound and/or presenting amplified sound to user 131 via an in-ear receiver. Processor 104 may be implemented by any suitable combination of hardware and software.

As shown in FIG. 1, hearing device 101 may further include an audio input unit 113 and an audio output unit 117 communicatively coupled to processor 104. Audio input unit 113 is configured to obtain an input audio stream. Processor 104 is configured to provide for a processing of the audio stream to obtain an output audio stream. Audio output unit 117 is configured to output sound based on the output audio stream.

In some implementations, as illustrated, audio input unit 113 may comprise a sound detector 115 configured to detect sound in an ambient environment of the user and to provide an ambient audio stream representative of the detected sound. The audio stream, which is received by processor 104, may then at least partially be based on the ambient audio stream. In some examples, sound detector 115 may be implemented as a microphone and/or a microphone array. In some examples, after detection of the sound in the ambient environment, audio input unit 113 may be configured to prepare the ambient audio stream for an audio signal processing by processor 104. For example, audio input unit 117 may comprise an analog-to-digital converter to convert the ambient audio stream, as detected by sound detector 115, from an analog signal into a digital signal.

In some implementations, as illustrated, audio input unit 113 may comprise a radio receiver 116 configured to receive a radio audio stream from a remote audio source via radio frequency (RF) radiation. The audio stream, which is received by processor 104, may then at least partially be based on the radio audio stream. Radio receiver 116 may be configured for wireless data reception of the radio audio stream. For instance, the radio audio stream may be received in accordance with a Bluetooth^{™} protocol and/or by any other type of RF communication. In some examples, the remote audio source may be a remote microphone, e.g., a table microphone or a clip-on microphone, configured to detect sound at a remote location and transmit the radio audio stream representative of the detected sound to radio receiver 116. In some examples, the remote audio source may be a streaming source, e.g., a media streaming source, configured for streaming the radio audio stream to radio receiver 116. In some examples, the remote audio source may be a communication device, e.g., a portable device such as a smartphone, tablet, smartwatch and/or the like, or a computing device such as a personal computer, configured for data transmission of the radio audio stream to radio receiver 116. In some examples, after reception of the radio audio stream, audio input unit 113 may be configured to prepare the radio audio stream for an audio signal processing by processor 104. For example, when the radio audio stream received from the remote audio source comprises an encoded signal, audio input unit 113 may comprise a decoder to decode the radio audio stream.

Audio output unit 117 may be implemented by any suitable audio output device configured to output sound based on the output audio signal to the user. To this end, audio output unit 117 may include an output transducer. For example, audio output unit 117 may be implemented as a receiver of a hearing aid, a loudspeaker of an earbud, or an output electrode of a cochlear implant.

Hearing device 101 may include further components as may serve a particular implementation. E.g., hearing device 101 may further include a user interface and/or a communication port for data transmission and/or an ear-canal microphone.

In some examples, hearing device 101 may include one or more sensors 175 in addition to sound detector 115 and/or RF receiver 116, which may also be denoted as one or more auxiliary sensors. The additional sensor 175 may provide additional, e.g., auxiliary, sensor data, which may be received by processor 104. In some examples, the auxiliary sensor may include a motion sensor and/or a physiological sensor. The motion sensor may be any suitable sensor configured to provide motion data indicative of a movement of a user wearing the hearing device. In some examples, the motion sensor can include an accelerometer and/or a gyroscope and/or a magnetometer and/or an inertial measurement unit (IMU). The physiological sensor may be any suitable sensor configured to provide physiological data indicative of a physiological property of the user. In some examples, the physiological sensor comprises an optical sensor, e.g., a photoplethysmography (PPG) sensor configured to detect a blood property. In some examples, the physiological sensor comprises a bioelectric sensor comprising at least one electrode configured to detect a bioelectric signal, e.g, an electrocardiogram (ECG) sensor and/or an electroencephalogram (EEG) sensor and/or an electrooculography (EOG) sensor. In some examples, the physiological sensor comprises a skin impedance sensor and/or a body temperature sensor.

FIG. 2 illustrates an exemplary implementation of hearing device 101 as a RIC hearing aid 161. RIC hearing aid 161 comprises a BTE part 170 configured to be worn at an ear at a wearing position behind the ear, and an ITE part 180 configured to be worn at the ear at a wearing position at least partially inside an ear canal of the ear. BTE part 170 comprises a BTE housing 171 configured to be worn behind the ear. BTE housing 171 accommodates a processing unit 164, which may comprise processor 104 and memory 102, communicatively coupled to sound detector 115 and radio receiver 116. BTE part 170 further includes an auxiliary sensor 175, e.g., a motion sensor and/or a physiological sensor, communicatively coupled to processing unit 164. BTE part 170 further includes a battery 177 as a power source. ITE part 180 is an earpiece comprising an ITE housing 181 at least partially insertable into the ear canal. ITE housing 181 accommodates audio output unit 117 implemented as a receiver. BTE part 170 and ITE part 180 are interconnected by a cable 174. Processing unit 164 is communicatively coupled to audio output unit 117 of ITE part 180 via cable 174 and cable connectors 172, 173 provided at BTE housing 171 and ITE housing 181.

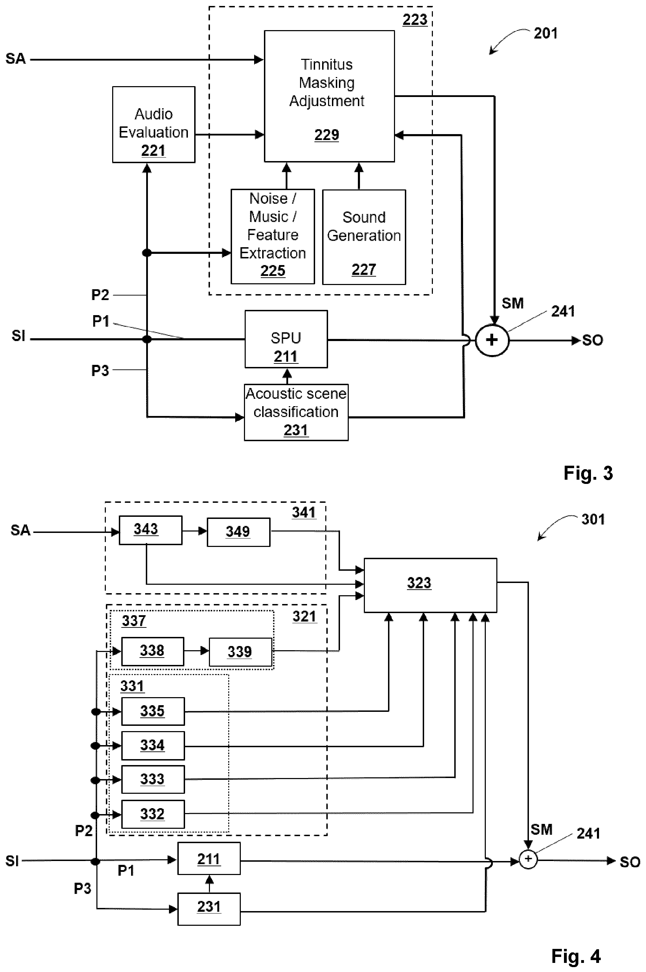

FIG. 3 is a schematic block diagram of a signal processing algorithm 201 for a processing of an audio stream SI so as to provide for relieving tinnitus. Algorithm 301 may be executed by processor 104 after receiving audio stream SI from audio input unit 113 as an input audio stream. Processor 104 may process the received audio stream to obtain an output audio stream SO, which may be output by audio output unit 117 to stimulate the user's hearing. In some examples, as illustrated, algorithm 201 may further receive auxiliary sensor data SA, e.g. motion data and/or physiological data, which may be provided by auxiliary sensor 175.

Algorithm 201 comprises a first audio signal path P1 for processing input audio stream SI into a processed audio stream based on which output audio stream SO is provided. First path P1 may also be referred to as a main path, or forward path. First path P1 is associated with applying one or more operations to audio stream SI. To this end, a signal processing unit (SPU) 211 is provided at first path P1. Operations performed by SPU 211 comprise an amplification operation for amplifying audio stream SI according to a transfer function.

In some examples, the transfer function may be customized, e.g., by adjusting one or more parameters of the transfer function, to match specific needs of an individual user, e.g., to compensate for an individual hearing loss of the user. In particular, by customizing the transfer function, hearing device 101 may be fitted to the specific needs and/or the individual hearing loss of user 131. In some examples, the fitting of hearing device 101 may comprise a step in which the transfer function is customized based on a measurement of a hearing loss of the user, e.g., based on an audiogram.

In some examples, the transfer function may comprise a gain model. E.g., the transfer function may at least partially be represented by the gain model. The gain model may define an amplification of audio stream SI provided at different frequencies and/or at different signal levels. E.g., the gain model may include one or more parameters defining a frequency-specific gain. The gain model may further include one or more parameters defining how the gain changes with input level, e.g., compression ratios and/or mappings of different input levels to output levels.

Algorithm 201 comprises a second audio signal path P2 for processing audio stream SI so as to output a tinnitus masking stream SM for relieving tinnitus into first path P1. Second path P2 may also be referred to as a side path, or parallel path, which may at least partially bypass first path P1. Second path P2 is associated with evaluating, in an audio stream evaluation operation, the audio stream SI with regard to a presence of one or more preservable characteristics suitable to be preserved in the tinnitus masking stream. To provide for the audio stream evaluation operation, an audio stream evaluation module 221 is provided at second path P2.

Second path P2 is further associated with providing, in a tinnitus masking stream provision operation, the tinnitus masking stream based on evaluated audio stream SI, e.g., based on the preservable characteristics determined to be present in audio stream SI, such that one or more of the preservable characteristics of the audio stream are preserved in the tinnitus masking stream. To provide for the tinnitus masking stream provision operation, a tinnitus masking stream provision module 223 is provided at second path P2.

To illustrate, characteristics of audio stream SI which may be suitable to be preserved in tinnitus masking stream SM may comprise a noise and/or a music content in audio stream SI. Audio stream evaluation operation 221 may then comprise determining whether the noise and/or music content is contained in audio stream SI. Tinnitus masking stream provision operation 223 may then comprise including the noise and/or music content in tinnitus masking stream SM. In some examples, the noise and/or music content may be extracted from audio stream SI, e.g., in a case in which audio stream SI contains also other signal components, so that it can be included in tinnitus masking stream SM. To this end, tinnitus masking stream provision module 223 may include an extraction module 225 configured to extract the noise and/or music content from audio stream SI.

To further illustrate, preservable characteristics of audio stream SI may also comprise any other signal feature of audio stream SI to be preserved in tinnitus masking stream SM. Audio stream evaluation operation 221 may then comprise determining whether such a signal feature is present in audio stream SI and/or determining one or more characteristics of the signal feature which are required for preserving the signal feature in tinnitus masking stream SM. Tinnitus masking stream provision operation 223 may then comprise a modifying of the tinnitus masking sound such that one or more of the signal features of audio stream SI are preserved, e.g., reproduced, in the tinnitus masking sound. E.g., the feature may be extracted from audio stream SI by extraction module 225.

Some examples of the signal feature of audio stream SI may include a timbre and/or a pitch and/or a beat and/or a rhythm of a sound represented by audio stream SI. Timbre may refer to the quality and/or color of the sound which makes the sound distinguishable from other sounds which may have the same pitch and/or loudness. E.g., timbre may be influenced by factors like harmonic content, attack and decay characteristics, and how the sound resonates. Pitch may refer to one or more frequencies of the sound. E.g., pitch may indicate a perceived frequency and/or a primarily enhanced frequency, which may also be influenced by other factors like loudness or the user's hearing ability. Rhythm may refer to any pattern, e.g., any temporally recurring characteristic, of sound and/or silence in audio stream SI. For instance, the rhythm may indicate a temporal characteristic of the pattern, e.g., a frequency of occurrence in audio stream SI. Similarly, a beat may refer to any temporally recurring characteristic in the sound. For instance, the beat may be representative of the recurring characteristic so as to reproduce the recurring characteristic, e.g., a periodic variation of the amplitude.

In some examples, tinnitus masking stream provision operation 223 comprises generating, in a tinnitus masking sound generation operation, a tinnitus masking sound different from audio stream SI. To this end, tinnitus masking stream provision module 223 may comprise a masking sound generation module 227. E.g., module 227 may be configured to generate the masking sound based on an artificial noise generated by a noise generator and/or a predetermined masking sound stored in a sound library.

In some examples, the tinnitus masking sound is generated depending on an input of user 131. E.g., the user may select a tinnitus masking sound from the sound library and/or indicate preferences with regard to properties of the generates noise. In some examples, the tinnitus masking sound is randomly generated, e.g., randomly selected from the sound library and/or randomly generated by the noise generator. In some examples, the tinnitus masking sound is generated depending on audio stream evaluation operation 221.

In some examples, before the masking sound generated by module 227 is included in tinnitus masking stream SM, the generated tinnitus masking sound is modified such that one or more of the preservable characteristics of audio stream SI are preserved in the tinnitus masking sound. The preservable characteristics may then comprise one or more of the signal features of audio stream SI, e.g., the timbre and/or pitch and/or beat and/or rhythm of sound represented by audio stream SI. To illustrate, after determining the signal feature of audio stream SI in audio stream evaluation operation 221 and during or after generating the tinnitus masking sound in masking sound generation operation 227, a timbre and/or pitch and/or beat and/or rhythm of the generated masking sound may be adjusted to the timbre and/or pitch and/or beat and/or rhythm of the sound in audio stream SI. By including the adjusted generated masking sound in tinnitus masking stream SM, tinnitus masking stream SM can thus be assimilated to sound contained in audio stream SI.

In some examples, tinnitus masking stream provision operation 223 comprises adjusting, in a tinnitus masking stream adjustment operation, tinnitus masking stream SM depending on audio stream SI evaluated in audio stream evaluation operation 221. To this end, tinnitus masking stream provision module 223 may comprise a tinnitus masking stream adjustment module 229. E.g., the adjustment may be performed on the noise and/or music content from audio stream SI, which may be provided by extraction module 225, and/or in the generated tinnitus masking sound, which may be provided by masking sound generation module 227.

Adjusting tinnitus masking stream SM may comprise modifying the noise and/or music content from audio stream SI before the including in tinnitus masking stream SM, e.g., by adjusting a property of the noise and/or music content. In some examples, the property comprises an amount and/or regularity and/or level of the noise and/or music content. In some examples, the property may be increased in tinnitus masking stream SM relative to the audio stream SI, e.g., depending on the evaluated audio stream SI.

Adjusting tinnitus masking stream SM may comprise mixing the noise and/or the music content from audio stream SI and the generated tinnitus masking sound, e.g., before and/or after the generated masking sound is modified with regard to the preservable characteristics of audio stream SI, in tinnitus masking stream SM. E.g., a mixing ratio of the mixing may be determined depending on audio stream SI evaluated in audio stream evaluation operation 221.

In some examples, audio stream evaluation operation 221 comprises evaluating audio stream SI with regard to one or more adjustment characteristics representative of an adjustment of tinnitus masking stream SM required when the preservable characteristics of audio stream SI are preserved in tinnitus masking stream SM. Adjusting tinnitus masking stream SM in operation 229 may then comprise providing tinnitus masking stream SM depending on the adjustment characteristics of evaluated audio stream SI, e.g., such that the preservable characteristics are adjusted accordingly in tinnitus masking stream SM.

In some examples, the adjustment characteristics are representative of a scaling of the preservable characteristics of audio stream SI, e.g., an amount to which the noise and/or music content from audio stream SI needs to be scaled and/or an amount to which the generated masking sound needs to be scaled before the including in tinnitus masking stream SM. E.g., when the noise and/or the music content from audio stream SI and the generated tinnitus masking sound are mixed according to a mixing ratio, the mixing ratio may be defined by the scaling represented by the adjustment characteristics. The adjustment characteristics may then also be referred to as scaling characteristics.

In some examples, the adjustment characteristics comprise a signal to noise ratio and/or a temporal variation and/or a level of a sound represented by audio stream SI. E.g., a rather low signal to noise ratio may indicate that an adjustment of the preservable characteristics of audio stream SI is required which prevents an even lower signal to noise ratio in the tinnitus masking stream SM. E.g., a rather large temporal variation may indicate that an adjustment of the preservable characteristics of audio stream SI is required which reduces the temporal variation in the tinnitus masking stream SM to enhance the tinnitus relieving effect. E.g., a rather low level may indicate that an adjustment of the preservable characteristics of audio stream SI is required which increases the level in tinnitus masking stream SM to enhance the tinnitus relieving effect.

In some examples, the adjustment characteristics comprise a presence and/or a type and/or an amount and/or a regularity and/or a level of speech represented by audio stream SI. To illustrate, when speech is contained in audio stream SI, it may be assumed that speech comprehension is of higher priority for the user than an optimization of the tinnitus relieving effect. Accordingly, the adjustment of the preservable characteristics of audio stream SI may be balanced such that speech comprehension is ensured and the tinnitus masking stream SM provides for a tinnitus relief which may be customized to these requirements and/or optimized under these conditions.

In some examples, the adjustment characteristics comprise a presence and/or a type and/or an amount and/or a regularity and/or a level of noise and/or music represented by audio stream SI. To illustrate, some types of noise and/or music in audio stream SI may be more suitable to provide for tinnitus relieving effect than others. Accordingly, those types may be predominantly selected in the adjustment of the preservable characteristics. Further, an amount and/or level of the noise and/or music may be enhanced in the adjustment of the preservable characteristics, e.g., when the enhancement yields a better relief effect and/or a speech comprehension would still be ensured. Further, a regularity of the noise and/or music may be enhanced in the adjustment of the preservable characteristics, e.g., also to provide for a better tinnitus relief.

In some examples, the adjustment characteristics comprise an acoustic scene which may be attributed to audio stream SI by an acoustic scene classifier 231. In particular, acoustic scene classifier 231 may also provide the acoustic scene to SPU 211, which may control one or more of the operations of the processing of audio stream SI in first path P1 depending thereon. In particular, different sound processing programs and/or actuators may be controlled and/or steered by SPU 211 depending on the acoustic scene attributed to audio stream SI. Audio stream evaluation operation 221 may then also include the acoustic scene classification performed by acoustic scene classifier 231. Accordingly, the adjustment of the preservable characteristics can be provided such that tinnitus masking stream SM is optimized for the current acoustic scene and/or optimized with regard to a sound processing programs and/or actuator executed in first path P1 by SPU 211.

In some examples, tinnitus masking stream adjustment module 229 may further receive auxiliary sensor data SA. Auxiliary sensor data SA may be based on sensor data provided by an auxiliary sensor. E.g., the auxiliary sensor may comprise motion sensor 175 and/or a physiological sensor. Tinnitus masking stream adjustment operation 229 may then be performed further depending on auxiliary sensor data SA, e.g., motion data and/or physiological data.

Algorithm 201 may further comprise a signal combiner 241 at path P1. Signal combiner 241 can be configured to combine, in a signal combination operation, the audio stream SI processed in path P1 with tinnitus masking stream SM, which is output from path P2 into path P1. E.g., SPU 211 may be provided preceding and/or subsequent to signal combiner 241. Some operations performed by SPU 211 may then be executed before the signal combination and/or some operations performed by SPU 211 may be executed after the signal combination. Output audio stream SO may be based on the combined stream. In some examples, the combining represents a mixing of audio stream SI processed in path P1 and masking stream SM processed in path P2 according to a mixing ratio. E.g., the mixing ratio may be determined depending on audio stream SI evaluated in audio stream evaluation operation 221 and/or depending on an input by user 131.

In some examples, tinnitus masking stream adjustment module 229 may be implemented as a trained machine learning (ML) algorithm, e.g., a DNN. E.g., the ML algorithm may be trained based on training data provided by audio stream evaluation module 221. In some examples, tinnitus masking stream provision module 223 may be implemented as a trained machine learning (ML) algorithm, e.g., a DNN. The ML algorithm may then be further configured to provide for noise and/or music and/or feature extraction operation 225. E.g., the noise and/or music may be separated from audio stream SI and/or audio stream SI may classified with regard to one or more characteristics of the feature. The ML algorithm may also be configured to provide for masking sound generation operation 227, e.g., the generate the masking sound depending on audio stream evaluation operation 221 and/or in a randomized manner and/or depending on an input by user 131.

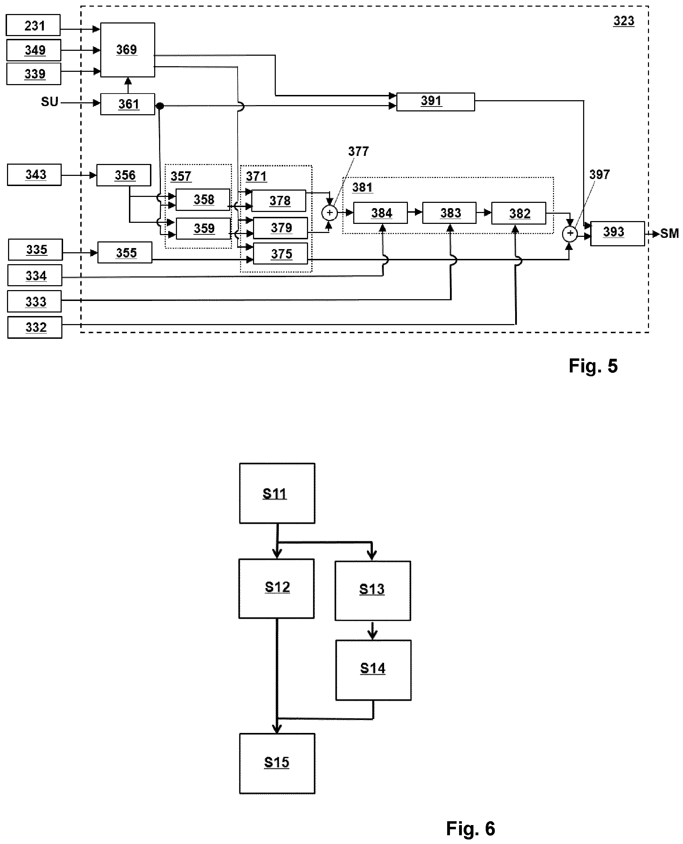

FIG. 4 is a schematic block diagram of another signal processing algorithm 301 for a processing of an audio stream SI so as to provide for relieving tinnitus. Algorithm 301 comprises a tinnitus masking stream provision module 323 which is further detailed in FIG. 5. Algorithm 301 further comprises an audio stream evaluation module 321 and an auxiliary sensor data evaluation module 341.

Audio stream evaluation module 321 comprises a preservable characteristics evaluator 331 which can evaluate audio stream AI with regard to a presence of one or more preservable characteristics suitable to be preserved in the tinnitus masking stream SM. Preservable characteristics evaluator 331 may comprise signal feature determining modules 332, 333, 334. Modules 332 - 334 are each configured to determine a specific feature in audio stream SI and/or characteristics of the specific feature representing characteristics of audio stream SI suitable to be preserved in tinnitus masking stream SM. In the illustrated example, module 332 is implemented as a timbre detector configured to determine a timbre of sound in audio stream SI and/or characteristics thereof. Module 333 is a pitch detector configured to determine a pitch of sound in audio stream SI and/or characteristics thereof. Module 334 is a beat and/or rhythm detector configured to determine a beat and/or rhythm of sound in audio stream SI and/or characteristics thereof. Preservable characteristics evaluator 331 may also comprise a noise determining module 335. Module 335 can be configured to determine one or more noise characteristics of audio stream SI, e.g., a presence and/or a type and/or an amount and/or a regularity and/or a level of noise contained in audio stream SI. In some examples, audio stream evaluation module 321 may further comprise a music determining module, which may be configured to determine one or more noise characteristics of audio stream SI, e.g., a presence and/or a type and/or an amount and/or a regularity and/or a level of music represented by audio stream SI.

Audio stream evaluation module 321 further comprises an adjustment characteristics evaluator 337 which can evaluate audio stream SI with regard to one or more adjustment characteristics representative of an adjustment of tinnitus masking stream SM. Adjustment characteristics evaluator 337 may comprise an acoustical environment analyzer 338 which may analyze audio stream SI with regard to one or more characteristics of audio stream SI indicative of the acoustic environment of user 131. In some examples, the characteristics of the acoustic environment of user 131 may be based on a classification of audio stream SI performed by a sound classifier 339. E.g., the classification may indicate a proportion of audio stream SI related to a quiet environment, a proportion of audio stream SI related to a noisy environment, a volume (or level) of the acoustic environment, e.g., relative to a speech contained in audio stream SI, a volume (or level) of the speech, a signal to noise ratio, e.g., of speech relative to noise in the environment, a temporal variation of sound in audio stream SI, a proportion of audio stream SI related to a music played in the environment, a regularity of noise in the environment, and/or the like.

Auxiliary sensor data evaluation module 341 may further receive auxiliary sensor data SA to evaluate, in an auxiliary sensor data evaluation operation, sensor data SA with regard to a presence of one or more adjustment characteristics representative of an adjustment of tinnitus masking stream SM required when the preservable characteristics of audio stream SI are preserved in tinnitus masking stream SM. Adjusting tinnitus masking stream SM in the tinnitus masking stream adjustment operation may thus depend on the adjustment characteristics of audio stream SI and/or the adjustment characteristics of auxiliary sensor data SA. E.g., auxiliary sensor data SA may comprise motion data provided by motion sensor 175 and/or physiological sensor data provided by a physiological sensor. Module 341 may comprise an auxiliary sensor data analyzer 343 which may analyze auxiliary sensor data SA with regard to indications which would require an adjustment of the preservable characteristics in tinnitus masking stream SM. In some examples, the indications in auxiliary sensor data SA may be based on a classification of auxiliary sensor data SA performed by a sensor data classifier 349.

In some examples, when auxiliary sensor data SA comprises motion data, classifier 349 may be implemented as a motion data classifier. E.g., the classification may indicate any physical activity, a resting state, a sleeping state, a standing or sitting or lying position, a walking or running or cycling activity, and/or the like. In some examples, when auxiliary sensor data SA comprises physiological data, classifier 349 may be implemented as a physiological data classifier. For instance, when the physiological data comprises EEG data, the classification may be performed with regard to different indications in the EEG data of an occurrence and/or a degree of a current tinnitus symptoms perceived by the user.

Tinnitus masking stream provision module 323, as illustrated in FIG. 5, comprises a noise extraction module 355 configured to extract noise from audio stream SI depending on the noise characteristics of audio stream SI determined by noise determining module 335. The extracted noise may then be included in tinnitus masking stream SM. Alternatively or additionally, module 323 may comprise a music extraction module configured to extract music from audio stream SI which may then be included in tinnitus masking stream SM.

Module 323 further comprises a masking sound generation module 357 for generating a tinnitus masking sound different from audio stream SI. Module 357 can comprise a noise generator 358 for generating the masking sound as artificial noise and/or a masking sound retrieving module 359 which may retrieve a predetermined masking sound stored in a sound library. E.g., the sound library may be stored in memory 102 of hearing device 101 and/or in a remote device, e.g., a mobile phone or other portable device, communicatively coupled to hearing device 101. The generated masking sound may then be included in tinnitus masking stream SM.

In some examples, the tinnitus masking sound may be randomly generated. E.g., noise generator 358 may generate the artificial noise with randomized characteristics and/or masking sound retrieving module 359 may randomly select and/or retrieve the masking sound from a plurality of different sounds stored in the sound library and/or modify characteristics of the retrieved masking sound in a randomized manner. In some examples, the random generation of the tinnitus masking sound may be based on a random number generated by a random number generator 356. In some examples, random number generator 356 is configured to determine the random number from auxiliary sensor data SA. E.g., number generator 356 may receive input from auxiliary sensor data analyzer 343 and/or may directly receive auxiliary sensor data SA as raw data to generate the random number based thereon. To illustrate, when auxiliary sensor data SA comprises motion data from a motion sensor, the random number may be generated based on the raw motion data, e.g., based on random movements performed by the user, and/or based on an evaluation of the motion data of auxiliary sensor data analyzer 343. In this way, the tinnitus masking sound may be generated by masking sound generation module 357 based on the motion data.

Module 323 further comprises a tinnitus masking stream control module 361 configured to receive a control signal SU. In some examples, control signal SU can be based on an input by user 131. E.g., the user input may be entered via a user interface which may be provided on hearing device 101 and/or a remote device communicatively coupled to hearing device 101, e.g., a mobile phone or other portable device. Control module 361 can be configured to provide for a customization of tinnitus masking stream SM depending on control signal SU.

In some examples, the tinnitus masking sound may be generated by masking sound generation module 357 based on control signal SU. E.g., control module 361 may control masking sound retrieving module 359 to select and/or retrieve the masking sound from the sound library based on control signal SU. To illustrate, user 131 may select a preferred masking sound from the sound library via the user interface which may be indicated by control signal SU. E.g., control module 361 may control noise generator 359 to generate the artificial noise with characteristics defined by control signal SU. For example, user 131 may select artificial noise characteristics according to his preferences via the user interface which may be indicated by control signal SU.

Module 323 further comprises a tinnitus masking adjustment decision module 369. Decision module 369 can be configured to provide, in the tinnitus masking stream adjustment operation, for the adjustment of tinnitus masking stream SM depending on the evaluated audio stream SI and/or depending on the evaluated auxiliary sensor data SA, e.g., depending on the adjustment characteristics of audio stream SI and/or the adjustment characteristics of auxiliary sensor data SA. To this end, decision module 369 may receive, from acoustic scene classifier 231 and/or sound classifier 339 and/or auxiliary sensor data classifier 349, information representative of the adjustment characteristics. In some examples, decision module 369 may be implemented by an ML algorithm. In some examples, decision module 369 may be implemented as a rule-based algorithm, e.g., a decision table. The rule-based algorithm may define rules for the adjustment of tinnitus masking stream SM depending on the adjustment characteristics.

Module 323 further comprises a scaling module 371. Scaling module 371 is configured to provide for a scaling of noise extracted from audio stream SI and/or a scaling of the tinnitus masking sound generated by masking sound generation module 357. To this end, scaling module 371 comprises a noise scaler 375 configured to scale the noise extracted from audio stream SI by noise extraction module 355. Module 371 also comprises an artificial noise scaler 378 configured to scale the artificial noise generated by noise generator 358. Module 371 further comprises a retrieved masking sound scaler 379 configured to scale the masking sound retrieved from the sound library by masking sound retrieving module 359.

In some examples, the scaling performed by module 371 may be based on the evaluated audio stream SI and/or the evaluated auxiliary sensor data SA. Masking adjustment decision module 369 may then control noise scaler 375 and/or artificial noise scaler 378 and/or retrieved masking sound scaler 379 to provide for the scaling depending on the adjustment characteristics of audio stream SI and/or the adjustment characteristics of auxiliary sensor data SA. In particular, an amount of the scaling, e.g., a scaling factor, may be decided by adjustment decision module 369 based on the adjustment characteristics of audio stream SI and/or the adjustment characteristics of auxiliary sensor data SA. The adjustment characteristics may then also be referred to as scaling characteristics of audio stream SI and/or auxiliary sensor data SA.

In some examples, the scaling performed by module 371 may be based on the acoustic scene attributed to audio stream SI by acoustic scene classifier 231. Noise scaler 375 and/or artificial noise scaler 378 and/or retrieved masking sound scaler 379 may then be controlled to provide for the scaling depending on the current acoustic scene. The amount of the scaling may then be also decided by adjustment decision module 369, which may receive information about the current acoustic scene from acoustic scene classifier 231.

By the scaling of the noise generated by noise generator 358 and/or the masking sound retrieved by module 359 from the sound library, a mixing ratio between the generated noise and the retrieved masking sound can be defined when the generated noise and the retrieved masking sound are combined by a signal combiner 377. Further, by the scaling of the noise extracted from audio stream SI and/or the tinnitus masking sound generated by masking sound generation module 357, a mixing ratio between the extracted noise and the generated masking sound can be defined when the extracted noise and the generated masking sound are combined by a signal combiner 397.

Module 323 further comprises a sound feature preservation module 381. Module 381 is configured to preserve one or more signal features of audio stream SI in the tinnitus masking sound generated by masking sound generation module 357. To this end, module 381 is configured to modify the generated tinnitus masking sound according to one or more of the preservable characteristics of audio stream SI determined by signal feature determining modules 332, 333, 334. Module 381 comprises a timbre modifier 382 providing for an adjustment of a timbre in the generated masking sound according to the timbre of sound in audio stream SI determined by timbre detector 332. Module 381 comprises a pitch modifier 383 providing for an adjustment of a pitch in the generated masking sound according to the pitch of sound in audio stream SI determined by pitch detector 333. Module 381 comprises a beat and/or rhythm modifier 383 providing for an adjustment of a beat and/or rhythm in the generated masking sound according to the beat and/or rhythm of sound in audio stream SI determined by beat and/or rhythm detector 333.

Module 323 further comprises a signal to noise ratio (SNR) maintenance module 391. Module 391 is configured to adjust tinnitus masking stream SM in a manner that a prescribed and/or desired SNR is maintained in output audio stream SO. In this regard, tinnitus masking stream SM may be regarded as a noise component which adds to a noise of audio stream SI processed in first path P1 when tinnitus masking stream SM is output into first path P1. Module 323 may thus adjust tinnitus masking stream SM so as to maintain the signal of audio stream SI relative to the noise at first path P1 to a prescribed and/or desired degree when the additional noise component of tinnitus masking stream SM is added to first path P1. E.g., when the signal at first path P1 comprises a speech, the SNR may be maintained by module 391 such that comprehension of the speech for the user is prioritized. As another example, when the processing at first path P1 is provided so as to compensate for a hearing loss of the user, the SNR may be maintained such that compensation of the hearing loss is still provided for, at least to a desired degree.

Under these constraints, SNR maintenance module 391 can be configured to adjust tinnitus masking stream SM in a manner that the tinnitus relief effect is provided for to a desired and/or optimized degree when output audio stream SO is output to the user. To this end, module 323 comprises a tinnitus masking stream scaler 393 configured to scale tinnitus masking stream SM. E.g., the scaling may be performed before tinnitus masking stream SM is output to first path P1 at signal combiner 241. A mixing ratio between tinnitus masking stream SM and audio stream SI processed at first path P1 may thus be defined by the scaling.

SNR maintenance module 391 may receive input from tinnitus masking adjustment decision module 369. A degree of the scaling may thus be controlled by adjustment decision module 369. E.g., when acoustic scene classifier 231 and/or sound classifier 339 identifies speech in audio stream SI, module 391 may maintain the SNR in output audio stream SO to ensure speech comprehension. SNR maintenance module 391 may also receive input from control module 361 depending on control signal SU. E.g., control signal SU may indicate an SNR of output audio stream SO desired by the user and/or an amount of tinnitus relief desired by the user, which may be input by user 131 via a user interface. A degree of the scaling may thus also be controlled via a user input.

Subsequently, some decisions that may be performed by decision module 369 are exemplified for illustrative purposes in the form of a decision table. Decision module 369 may perform the decisions based on an acoustic scene determined by acoustic scene classifier 231 and/or adjustment characteristics of audio stream SI determined by sound classifier 339 and/or adjustment characteristics of auxiliary sensor data SA, e.g., motion data, determined by sensor data classifier 349. The table further shows preservable characteristics of audio stream SI which may be determined by preservable characteristics evaluator 331, and exemplary resulting compositions of tinnitus masking stream SM.

| **Acoustic scene** | **Adjustment characteristics of audio stream SI** | **Preservable characteristics of audio stream SI** | **Composition of tinnitus masking stream SM** |
|---|---|---|---|
| quiet environment | - low volume | sound feature (e.g., rhythm and/or timing and/or timbre) | generated masking sound (modified according to sound feature in audio stream SI) |
| | - low signal variation | | |
| speech in quiet environment | - speech | sound feature | generated masking sound (modified according to sound feature in audio stream SI) |
| | - large SNR | | |
| low noise environment | - low volume | - extracted noise | - extracted noise (scaled) |
| | | | and/or |
| | - small SNR | - sound feature | - generated masking sound (modified according to sound feature in audio stream SI) |
| low music in quiet environment | - low volume | - extracted music | - extracted music (scaled) |
| | - large signal variation | | and/or |
| | | - sound feature | - generated masking sound (modified according to sound feature in audio stream SI) |
| | - medium SNR | | |
| low music in low noise environment | - low volume | - extracted music | - extracted music (scaled) and/or |
| | - large signal variation | - extracted noise | - extracted noise (scaled) and/or |
| | - low SNR | - sound feature | - generated masking sound (modified according to sound feature in audio stream SI) |
| noisy environment (e.g., car) | - mid volume | - extracted noise | None |
| | - small SNR | - sound feature | |
| loud speech in low noise environment | - loud speech | - extracted noise | - extracted noise (scaled) and/or |
| | - large SNR | - sound feature | - generated masking sound (modified according to sound feature in audio stream SI) |
| speech in noise environment | - speech | - extracted noise | None |
| | - small SNR | - sound feature | |
| Talk on the phone | speech | None | None |
| Media streaming | media | None | None |
| sleep in quiet (e.g. bed) | - low volume | sound feature | - generated masking sound (modified according to sound feature in audio stream SI) |
| | - low signal variation | | |
| | - motion data: no movement | | |
| sleep with some noise (e.g. train, plane etc.) | - low volume | - extracted noise | - extracted noise (scaled) |
| | - small SNR | | and/or |
| | - motion data: no movement | - sound feature | - generated masking sound (modified according to sound feature in audio stream SI) |
| Deep Sleep | - slow regular breathing sound | sound feature | None |
| | - motion data: no movement | | |

FIG. 6 illustrates a block flow diagram for an exemplary method of processing an audio signal in a hearing device. At operation S11, audio stream SI is received. At operation S12, audio stream SI is processed in a first audio signal path into a processed audio stream. At operations S13 and S14, audio stream SI is processed in second path P2, which outputs tinnitus masking stream SM into first path P1. S13 is an audio stream evaluation operation in which audio stream SI is evaluated with regard to a presence of one or more preservable characteristics which are suitable to be preserved in tinnitus masking stream SM. S14 is a tinnitus masking stream provision operation in which tinnitus masking stream SM is provided based on the preservable characteristics such that one or more of the preservable characteristics of audio stream SI are preserved in tinnitus masking stream SM. At operation S15, audio output stream SO is provided based on the processed audio stream so that it can be output by audio output unit 117.

While the principles of the disclosure have been described above in connection with specific devices, systems and methods, it is to be clearly understood that this description is made only by way of example and not as limitation on the scope of the invention. The above described preferred embodiments are intended to illustrate the principles of the invention, but not to limit the scope of the invention. Various other embodiments and modifications to those preferred embodiments may be made by those skilled in the art without departing from the scope of the present invention that is solely defined by the claims. In the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A method of operating a hearing device to provide for relieving tinnitus, the hearing device configured to be worn at an ear of a user, the method comprising
receiving an audio stream (SI) from an audio input unit (113) included in the hearing device;
processing (211) the audio stream (SI) in a first audio signal path (P1) into a processed audio stream; and
providing an output stream (SO) based on the processed audio stream for an audio output unit (117) included in the hearing device, the audio output unit (117) configured to output the output stream (SO) so as to stimulate the user's hearing;
**characterized by**
processing the audio stream (SI) in a second audio signal path (P2), which outputs a tinnitus masking stream (SM) for relieving tinnitus into the first audio signal path (P1), wherein the second audio signal path (P2) is associated with
evaluating (221, 331) the audio stream (SI) with regard to a presence of one or more preservable characteristics suitable to be preserved in the tinnitus masking stream (SM); and
providing (223, 323) the tinnitus masking stream (SM) based on the preservable characteristics such that one or more of the preservable characteristics of the audio stream (SI) are preserved in the tinnitus masking stream (SM).

2. The method of claim 1, wherein the preservable characteristics comprise a noise and/or a music content in the audio stream (SI), the method further comprising
including the noise and/or the music content from the audio stream (SI) in the tinnitus masking stream (SM).

3. The method of claim 2, wherein the noise and/or music content from the audio stream (SI) is modified before the including in the tinnitus masking stream (SM).

4. The method of any of the preceding claims, further comprising
generating (227, 357) a tinnitus masking sound different from the audio stream (SI), wherein the generated tinnitus masking sound is included in the tinnitus masking stream (SM) depending on the evaluated audio stream (SI).

5. The method of claim 4, wherein the tinnitus masking sound is generated based on one or more of
an artificial noise generated by a noise generator (358); or
a predetermined masking sound stored in a sound library (359).

6. The method of claim 4 or 5, further comprising
receiving motion data (SA) from a motion sensor (175) included in the hearing device, wherein the tinnitus masking sound is generated based on the motion data (SA).

7. The method of any of claims 4 to 6, wherein, before the generated tinnitus masking sound is included in the tinnitus masking stream (SM), the generated tinnitus masking sound is modified (381) such that one or more of the preservable characteristics of the audio stream (SI) are preserved in the tinnitus masking stream (SM).

8. The method of claim 7, wherein the preservable characteristics of the audio stream (SI) preserved in the generated tinnitus masking sound include one or more of
a timbre of a sound represented by the audio stream (SI);
a pitch of a sound represented by the audio stream (SI);
a beat and/or rhythm of a sound represented by the audio stream (SI).

9. The method of claim 2 or 3 and any of claims 4 to 8, wherein the noise and/or the music content from the audio stream (SI) and the generated tinnitus masking sound are mixed in the tinnitus masking stream (SM) according to a mixing ratio, wherein the mixing ratio is determined depending on the evaluated audio stream (SI).

10. The method of any of the preceding claims, further comprising
evaluating (221, 337) the audio stream (SI) with regard to one or more adjustment characteristics representative of an adjustment of the tinnitus masking stream (SM) required when the preservable characteristics of the audio stream (SI) are preserved in the tinnitus masking stream (SM); and
adjusting (229, 371, 381, 391, 393) the tinnitus masking stream (SM) depending on the adjustment characteristics.

11. The method of claim 10, wherein the adjustment characteristics comprise one or more of
a signal to noise ratio of a sound represented by the audio stream (SI);
a temporal variation of a sound represented by the audio stream (SI);
a level of a sound represented by the audio stream (SI);
a presence and/or a type and/or an amount and/or a regularity and/or a level of speech represented by the audio stream (SI);
a presence and/or a type and/or an amount and/or a regularity and/or a level of noise represented by the audio stream (SI);
a presence and/or a type and/or an amount and/or a regularity and/or a level of music represented by the audio stream (SI); or
an acoustic scene attributed to the audio stream (SI) by an acoustic scene classifier (231), wherein one or more of the operations for the processing of the audio stream (SI) in the first audio signal (P1) path are controlled depending on the acoustic scene attributed to the audio stream (SI).

12. The method of any of the preceding claims, further comprising
receiving auxiliary sensor data (SA) from an auxiliary sensor (175) included in the hearing device;
wherein the processing the audio stream (SI) in the second audio signal path (P2) further comprises
evaluating (341) the auxiliary sensor data with regard to a presence of one or more adjustment characteristics representative of an adjustment of the tinnitus masking stream (SM) required when the preservable characteristics of the audio stream (SI) are preserved in the tinnitus masking stream (SM); and
adjusting (229, 371, 381, 391, 393) the tinnitus masking stream (SM) depending on the adjustment characteristics.

13. The method of claim 12, wherein the auxiliary sensor data (SA) comprises motion data from a motion sensor and the adjustment characteristics of the motion data comprise one or more of
a physical activity of the user;
a resting state of the user;
a sleeping state of the user;
a standing or sitting or lying position of the user; or
a walking or running or cycling activity of the user.

14. The method of any of the preceding claims, wherein the first audio signal path (P1) is associated with applying one or more operations to the audio stream (SI) comprising an amplification operation for amplifying the audio stream (SI) according to a transfer function.

15. A hearing device configured to be worn at an ear of a user, the hearing device comprising
an audio input unit (113) for obtaining an audio stream (SI);
a processor (104) for audio signal processing of the audio stream (SI) to obtain an output stream (SO); and
an audio output unit (117) for outputting the output stream (SO) so as to stimulate the user's hearing,
**characterized in that** the processor (104) is configured to perform the method of any of claims 1 to 14.
